# EUROPEAN PATENT SPECIFICATION

(11) **EP 4 210 783 B1**
(45) Date of publication and mention of the grant of the patent: **29.04.2026**
(21) Application number: 21765585.1
(22) Date of filing: 11.08.2021
(51) Int. Cl.: A61M 5/142

(54) **PATCH INJECTION DEVICE WITH AN IMPROVED RELEASE LINER REMOVAL**
PATCH-INJEKTIONSVORRICHTUNG MIT VERBESSERTER ENTFERNUNG DER ABZIEHFOLIE
DISPOSITIF D'INJECTION PATCH AYANT UN ENLÈVEMENT AMÉLIORÉ DE LA REVETEMENT

(30) Priority: 09.09.2020 EP 20195282
(43) Date of publication of application: 19.07.2023
(73) Proprietor: Ypsomed AG, 3401 Burgdorf (CH)
(72) Inventor: FIECHTER, Marc, 3510 Konolfingen (CH); BART, Serge, 3007 Bern (CH); BURREN, Stefan, 3150 Schwarzenburg (CH); MARBERT, Regina, 4933 Rütschelen (CH); SCHAERER, Balz, 3298 Oberwil bei Büren (CH)
(74) Representative: Meier Obertüfer, Jürg
(86) International application number: PCT/EP2021/072343
(87) International publication number: WO 2022/053248

(56) References cited:
- WO-A1-2010/029054
- WO-A1-2011/075101
- WO-A1-2018/081234
- US-A1- 2016 213 322

## Description

### FIELD OF THE INVENTION

The current invention relates to a patch injection device comprising a housing with an adhesive substrate connected thereto and the adhesive substrate is covered by a release liner. The adhesive substrate and the release liner are configured to improve the removal of the release liner from the patch injection device.

### BACKGROUND OF THE INVENTION

Injection or infusion devices are frequently used for the subcutaneous delivery of fluid medicaments. Infusion devices such as infusion pumps are used for delivery according to a delivery profile comprising a basal rate superimposed by bolus injections for medicaments such as insulin. Injection devices such as injection pens are used for single bolus type of injections according to a preset (fixed) dose or to a variable dose.

Patch injection devices are provided as a patch, i.e. can be adhered to the skin of the patient and can be operated for the delivery according to a delivery profile or for bolus type of injections only. The dose is delivered from a reservoir via a fluid path ending in a needle or cannula. The patch injection device is attached to the skin of the patient using a substrate containing a skin adhesive. Prior to use, the skin adhesive is covered by a release liner and the skin adhesive should allow for easy device removal after completion of the injection without tissue damage. Patch injectors may be designed for the delivery of large bolus volumes (large dose bolus injectors), comprising reservoirs with a volume above 1 mL, for example 5mL or 10 mL. In such case the weight of the patch injector may call for an improved skin adhesion system.

Removal of the release liner by the user is preferably done by holding the device in one hand and peeling the release liner by the other hand. Solutions where both hands are required, i.e. holding/peeling the substrate with one hand and holding/peeling the release liner with the other hand, may result in that the device itself is not held by the user. This may lead to dropping the device while peeling the release liner thereby potentially damaging the device or wasting the drug. This is harmful to the patients' therapy and moreover, an expensive medicament will be lost.

The substrate used for skin attachment is preferably a flexible substrate such that the substrate can accommodate to the contours of different types of patients and can be used at different anatomical locations on the body requiring a different shape of the injection device that a (rigid) housing may not provide.

WO17219154 describes a patch injection device having a substrate for skin attachment and the substrates' surface area equals the bottom surface area of the housing whereas the release liner extends beyond the substrate forming a pull tab. The user holds can hold the device in one hand and grab the extended area of the release liner with his other hand for liner removal. The maximum size of the cartridge (or weight of the device) may be restricted by the skin adhesive used combined with the limited substrate surface area available for skin contact.

WO07122207 discloses a modular patch injection device with an adhesive substrate extending beyond the device housing. The release liner covers the adhesive and no pull tab is provided for removing the release liner. Liner removal is cumbersome as both hands need to be used to grab both the substrate and the liner and no hand will be free for holding the device housing.

WO19008562 shows an arrangement for a patch injection device using two separate liners, e.g. both hands are needed for removing both liners at once.

In EP2914314 the substrate comprising the skin adhesive forms a skirt surrounding the device. The substrate is flexible and comprises a separate stiffener which may be stiff enough to resist a force on the substrate caused by peeling of the cover (release liner).

US20160213322 discloses a patch injection device with an adhesive substrate surrounding a housing, the substrate being covered by a release liner having a pull tab extending from a cut-out of the adhesive substrate.

It is an objective of the present invention to provide an improved patch injection device with an improved device handling overcoming the disadvantages of the prior art in combining an increased contact surface for skin attachment with an improved release liner removal.

This objective is solved by the independent claim 1. The patch injection device comprises an adhesive substrate for skin attachment and the adhesive substrate defines a skirt surrounding the housing of the patch injection device. The substrate comprises a cut out whereas the release liner extends beyond the cut out such that the release liner can peeled by the user while holding the device. The cut-out extends to the housing of the device such that the peel off force is directly transmitted or guided to the housing.

### DEFINITIONS

The term "medicament" or "medication" includes any flowable medical formulation suitable for controlled administration through a means such as, for example, a cannula or a hollow needle and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from -or harvested by- biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

The distal end or distal direction is defined by the direction of the needle configured to penetrate the skin of the patient. For a patch injection device the distal end and the distal direction is towards the needle configured to penetrate the skin of the patient, which may be along the axis of the device or tilted or perpendicular to the axis of the device. The proximal direction or end is opposite to the distal direction or end.

In the claims, the word "comprising" does not exclude other elements or steps, and the indefinite article "a" or "an" does not exclude a plurality. For example "substrate", "liner" or "adhesive" does not exclude the fact that there may be two "liners", "substrates" or "adhesives" that functionally or structurally fulfill the purpose of "a liner" or "a substrate" or "an adhesive".

### GENERAL DESCRIPTION OF THE INVENTION

The current invention relates to a patch injection device comprising a housing and an adhesive substrate having a first surface with a first surface area for attachment to the skin and a second surface which is opposite the first surface attaching at least partly to the housing. The patch injection device comprises a removable release liner. The surface area of the second surface attaching to the housing is smaller than the first surface area and the excess area defines a skirt of the substrate which at least partially surrounds the housing. The first surface of the substrate attaches to the removable release liner which fully covers the first surface area of the substrate. The patch injection device furthermore comprises a pull tab that is preferably part of the release liner and which is located in a cut out of the skirt of the substrate such that a peel force is transmittable from the release liner to the housing at the location where the cut-out reaches the housing. In other words, the skirt of the substrate surrounding the housing is interrupted at the location of the pull tab, and presents or includes a cut out or recess in the substrate of a width preferably at least equal to the width of a thumb of the patient.

The skirt at least partially surrounding the housing extends the area of the adhesive substrate beyond the area of the housing, and the excess area improves the attachment to the skin such that a less strong skin adhesive may be used thus improving the desired removal of the device from the skin after use. Alternatively larger volume reservoirs or cartridges (for example 5 mL or 10 mL) may be used as the latter define a substantial part of the weight of the device and the excess area avoids undesired detachment of the injection device before completion of the injection. As an alternative both an easy to remove skin adhesive and a large volume medicament reservoir may be used in combination with a skirt surrounding the housing.

The removable release liner is formed as a sheet that at least fully covers the first surface area of the adhesive substrate and prevents undesired attachment of the patch injection device prior to use. The **pull** tab is preferably formed, like the release liner, as a sheet and improves the removal of the release liner from the patch injection device. The pull tab is part of the release liner, or at least connected to the release liner in a manner to transmit to the release liner a release force applied to the **pull** tab, and possibly reinforced by additional layers.

The location of the **pull** tab in a cut out of the skirt ensures that the **pull** tab locally extends beyond the skirt and can be easily grabbed by the user on both sides for liner removal. The cut out in the skirt of the substrate ensures that the **pull** tab is free standing and locally extending beyond the skirt or the housing of the device thus leaving both surfaces of the sheet forming the **pull** tab available for a user to remove the release liner.

The peel force is transmittable from the release liner to the housing where the cut out reaches the housing. This implies that the peel force is locally directly transmitted to the housing where the substrates' cut out is adjacent to the housing and the peel force is not transmitted to the substrate or part of the substrate that is not adjacent to the housing. The latter part of the substrate (not adjacent to the housing) may be damaged, flexed or is compliant and the release of the release liner may require both hands of the user to peel the substrate from the release liner and/or peel the release liner from the substrate. The peel force is directly transmitted to the housing, which is held by the user in one hand as the user grabs the **pull** tab with his other hand. The mechanical stress is locally concentrated at the interface between the release liner and the first surface of the substrate (being locally supported or backed-up by the rigid housing). The concentration of the mechanical stress forms a starting point for controlled liner removal such that the release liner can be easily removed in one piece without tearing the liner (or the substrate). The user can hold the device in one hand and the pull tab in the other hand. This in contrast to the prior art devices where two **pull** tabs, each connected to two parts of the release liner, must be held by the user using both hands and no hand will be free for holding the device which complicates handling, or requires more handling steps.

Alternatively, the patch injection device comprises two cut-outs in the substrate that each reach to the housing.

Two or more cut outs in the substrate may have the advantage that the device may be held in two different orientations for liner removal. As the device is held with one hand by the user, the two cut outs may be positioned to facilitate liner removal either for left handed or for right handed users.

The release liner partially extends beyond the adhesive substrate, preferably in the area of the cut out, such that the surface area of the release liner is larger than the first surface area.

Preferably, the release liner follows the outer contours of the adhesive substrate except for the part where there is a cut out in the substrate. The part of the release liner in the cut out of the substrate may therefore form a pull tab. The release liner preferably follows the virtual contour of the substrate as if there was no cut out. For example, the substrate may have a rectangular shape with one corner left out forming the cut-out. The release liner may have the full rectangular shape including all four corners and thus extends from the substrate in the area of the cut out.

The patch injection preferably comprises an adhesive substrate which is flexible. Flexible is defined as being non-rigid and capable to be compliant with a non-flat or curved surface. Flexible materials typically have a young's modulus below 1 GPa, more preferably below 0.2 GPa, most preferably below 0.1 GPa. Examples are sheets of paper, or thin film materials (below 0.3 mm thick) made from a polyester such as PET, a polyamide or a polyolefenic material.

A flexible substrate ensures that the substrate may follow the contours of the patient's body once adhered. Optionally only the skirt surrounding the housing is flexible and compliant with the shape of the patient's body. The flexibility of the substrate ensures a good contact to the body and efficient use of a body adhesive thereby preventing release of the patch injection device before completion of the injection. Alternatively, only the skirt area comprises a skin adhesive and the area of the first surface opposite to the second surface area contacting the housing has no skin adhesive to avoid excessive perspiration during use. Alternatively, two different types of skin adhesives or two different substrates may be used, one for the skirt and the other for the center area.

The pull tab may be integrally formed with the release liner or the pull tab is a separate part attached to the release liner.

The pull tab extends from the device, preferably from the cut out of the substrate for easy access to the user. The pull tab may be a separate part attached to the release liner such that the pull tab may extend even further from the device beyond the (virtual) contour of the substrate. The pull tab can be made from a different material with a higher tear strength, or the pull tab has a textured surface for example giving a different haptic feeling to the user. Optionally, the pull tab as a separate but attached part that may have a different color or may be used for branding the product. A separate pull tab may thus increase the versatility of the device. Optionally, the pull tab may be made from an elastomeric material whereas the release liner is made from a thermoplastic sheet. The pull tab as a separate part may be formed as a sleeve, for example the pull tab may be part of a needle cover sleeve.

Indicators such as arrows may be printed onto the pull tab both for the integrally formed and the separate pull tab.

The pull tab preferably extends in a plane that is parallel to the first surface or parallel to the substrate. Alternatively the pull tab extends in a plane that is angulated with respect to the first surface or is even perpendicular to the first surface.

The patch injection device wherein the pull tab comprises a hole. The hole may be formed in the sheet of the release liner when the pull tab is integrally formed with the release liner, or the hole may be in an extension of the separate part forming the pull tab, for example a pull ring.

A hole in the pull tab, independent from being integrally formed with the release liner or designed as a separate part, has the advantage that the tab can easily be gripped by the user to support liner removal. The hole may be circular or elliptical shaped, or have a different shape such as an arrow shape.

The patch injection device wherein the peel force of the release liner is transmittable to the housing via the adhesive substrate at the location where the cut out reaches the housing.

At the location where the cut-out reaches the housing, there is a border area where the housing and the substrate end, whereas the release liner continues to extend in the lateral direction (or parallel to the bottom surface of the housing). Peeling the release liner by pulling the pull tab will result in a direct load transfer from the release liner to the housing via the substrate. The preferably flexible substrate will locally not flex or bend as the substrate is locally mechanically supported by a mechanically stiff housing or housing part. Thus by using the cut out, flexing and deformation of the substrate during liner removal is avoided. Once the initial part of the release liner has been removed from the substrate adjacent to the cut out, the further removal will not be complicated as the peel-off force will be evenly distributed and the peel off starts from the defined starting point. This is particularly useful in combination with a flexible substrate.

Cut out reaches the housing is defined in that a part of the cut out exactly reaches (coincides with) the location where the second surface attaches to the housing or is adjacent to that location, either within the area of the skirt or within the second surface such that there is an overlap between the second surface and the cut-out.

The cut-out of the skirt in the substrate is preferably arc shaped. The cut out of the substrate may have different shapes such as an arc shape or an elliptical shape or a circular shape. The contour forming the shape is close to or adjacent to the housing. The release liner extending from the cut out may have indicators, for example an arrow, printed thereon for guiding the user into the peel direction.

Alternatively, the cut-out is semi circular shaped, S-shaped, C-shaped, or there is a linear edge for cut out. A linear cut-out may result in a triangular shaped cut out.

The preferably arc shaped cut-out of the skirt is adjacent or locally congruent to the contacting surface between the housing and the substrate. Thus the edge of the surface area of the second surface attaching to the housing is adjacent to the contour forming the cut-out.

The housing of the patch injection device comprises at least one corner and the at least one corner is adjacent to the arc shaped cut out of the skirt.

The corner of the housing is preferably a rounded corner and located in a plane parallel to the substrate. Alternatively the corner is located in a plane perpendicular to the substrate. As yet another alternative, the corner is rounded in both planes forming a cup shaped corner.

The patch injection device wherein the release liner contacting the substrate is split into two separate parts each having a separate pull tab.

Optionally, the adhesive substrate is partially stiffened, preferably in the skirt surrounding the housing. To further improve the removal of the release liner, especially in combination with a flexible substrate, a further mechanical support can be provided to the substrate, preferably the mechanical support is provided locally on the substrate that is adjacent to the cut out. Thus a variable stiffness substrate can be achieved by locally applying stiffeners. Those may be formed by ribs formed onto, or sheets additionally applied to the substrate. Alternatively the substrate itself is locally folded or structured to locally improve the stiffness.

The adhesive substrate preferably is a multilayered substrate comprising at least one supportive layer and at least two adhesive layers for attaching the housing to a part of the second surface and for attaching the removable release liner to the first surface.

The supportive layer may be a woven or non-woven (fleece) material, based on, for example, an organic material such as cotton. The supportive layer is preferably a flexible material. The open spaces in the woven material improve penetration and adhesion of the adhesive layers and are beneficial for the flexibility. Alternatively a plurality of supportive layers is used having internal adhesive layers between supportive layers and external adhesive layers for attachment to the release liner and housing, respectively. The skin adhesive layer may be based on an acrylic adhesive which is biocompatible. The internal layers may be electrically conductive or may have electrical contacts. For example conductive wires may be woven in the layer or conductive ink patterns (for example carbon or silver) may be printed onto the layers. Electrically conductive layers may be used as sensors within the substrate such as a capacitive sensor or proximity sensor or a temperature sensor.

The removable release liner may be made from a sheet of an organic material such as paper, or from a polymeric material for example polyethylene terephthalate (PET) or an olefinic polymer (Polypropylene or Polyethylene). The multilayered adhesive and/or the release liner substrate may have one or more apertures, for example allowing movement of an insertion needle from inside the housing to outside the housing.

The substrate may have another cut out, defined by the fact that the release liner does not extend therefrom for easy liner removal but, instead, the release liner follows the contour lines of this cut out. Such a cut out has a different purpose and is not related to the ease of liner removal but may be beneficial during assembly of the device.

Therefore a plurality of cut outs may be envisaged, either advantageous for liner removal (in combination with a release liner extending from the cut out) and/or advantageous for assembly.

The housing is an outside housing comprising a top housing part and a bottom housing part and the adhesive substrate is preferably attached to the bottom housing part.

Thus the second surface of the substrate is attached to the bottom housing part thereby forming the second surface area (attached to the housing) of the substrate. The bottom and top housing parts may be permanently attached to each other or the top housing part is releasably connected to the bottom housing part. The latter option may be beneficial for a semi-disposable device having a re-usable part (connected to or integrated with the top housing part) and a disposable bottom housing part connected to the substrate with the release liner. Part of the device is disposed after use and part of the device may be re-used for another medicament injection or infusion. The corners of the housing are at least located in the bottom housing part.

Optionally, the patch injection device may have a feature to facilitate the removal of the device from the skin after skin attachment. As the flexible skirt surrounding the housing completely attaches to the skin, it may be difficult to remove the device by the user. The patch injection device may have a feature to promote device removal.

The patch injection device may comprise a non-adhesive area in the substrate to promote removal of the patch injection device after skin attachment. The non-adhesive area being part of the first surface of the substrate. Preferably, the non-adhesive area is surrounded by adhesive for skin attachment and optionally, there is a gradual change from adhesive area to the non-adhesive area.

The non-adhesive area is preferably located in the skirt of the substrate forming a winglet having the advantage that the winglet is not attached to the skin and can easily be grasped for removal of the patch injection device from the skin. Optionally, the non-adhesive area or winglet is located adjacent from the pull tab of the release liner when the release liner is attached to the first surface of the substrate. The winglet is preferably located at a corner of the patch injection device.

The surface area of the non-adhesive area is less than 50% of the first surface area, preferably less than 20% more preferably less than 10% of the first surface area , most preferably 5% of the first surface area.

Optionally, the surface opposite to the non-adhesive area or the top surface of the winglet that is within the skirt comprises an indicator. The indicator may be textured for a haptic touch and/or may have a visual indicator. The visual indicator is, for example, an arrow indicating the direction of removal of the patch injection device. Additionally and optionally, the indicator may have a text message. Thus there may be an indicator on the release liner facilitating liner removal and there may be an indicator on the winglet for removal of the device form the skin. Optionally, the two indicators are adjacent to each other.

The patch injection device wherein the housing encloses a drive mechanism for expelling a medicament, a control unit for controlling medicament delivery and a fluid path comprising an injection needle for delivery of the medicament.

The drive mechanism may comprise an electric motor or the mechanism uses a different power package such as a spring or the mechanism is hydraulic driven or driven by (gas) pressure. The mechanism may comprise a piston rod for advancing a plug in a reservoir towards an outlet of the reservoir. A gear mechanism may be positioned between the electric motor and the piston rod converting rotational movement of the electric motor in linear movement of the piston rod. The piston rod may be a segmented piston rod and each segment being connected by a film link such that the piston rod can reversibly change from a linear to a curved configuration. The control unit may be based on a mechanical system, for example using motion links or, more preferably, the control unit is an electronic control unit located on a printed circuit board and comprising integrated circuits and a memory unit or a transmitter/receiver unit. The patch injection device comprises a battery when an electric motor and an electric control circuit is used.

The fluid path may comprise a flexible tubing connecting two insertion needles, one insertion needle for insertion into the skin of the patient and the other needle for insertion into the reservoir to establish a fluid connection between the reservoir and the skin insertion needle. The reservoir needle of the fluid path may be fixed in the housing and the reservoir is moved within the housing to establish the fluid connection or, alternatively, the needle is moved with respect to a reservoir that is fixed to the housing. This so called reservoir needle is moved by a reservoir needle insertion mechanism. The skin needle may be fixed to and extend from the housing and be covered by a moveable needle sleeve which is moved with respect to the housing for skin insertion. Alternatively, the skin needle is moveable arranged within the housing from a retracted position into an extended position outside the housing. The movement may be reversible or simply a one-way movement. A skin needle insertion mechanism inserts the skin needle and the needle insertion mechanism is preferably biased by a spring. The skin needle insertion mechanism may also provide the force for the needle retraction after the medicament has been delivered.

Furthermore, the housing of the patch injection device may house a cartridge comprising a fluid medicament. The cartridge may be a flexible reservoir or a rigid reservoir. Examples of a rigid reservoirs may be a glass or polymer cartridge (for example made from COC). The flexible or rigid reservoir may be closed by a pierceable septum configured to be pierced by the reservoir needle. Alternatively, the reservoir is permanently connected to the fluid path. A rigid cartridge may be closed by a plug or plunger such that the fluid medicament is enclosed between the plug and the septum. The cartridge may be present in the device having the advantage of a prefilled and ready to use device (but may restricted to single use). Alternatively, the cartridge is inserted into the device just prior to use. The cartridge may be inserted via a door mechanism comprising a cartridge holder or cover adapted to receive the cartridge. Optionally, the door mechanism comprises a cartridge needle for penetrating the septum when the cartridge is inserted into the door mechanism. As a further option, the door mechanism may comprise a latching mechanism which, once latched, mechanically or electrically unlocks the device from a dormant state into an active state (ready to use after filling). An advantage of insertion the cartridge just prior to use may be that delicate medicaments can be mixed or reconstituted outside the device just prior to use.

A patch injection device that is of the semi-disposable type comprises a disposable part having the bottom housing part with the adhesive substrate, the release liner and the components that may contact the fluid medicament such as the cartridge and the fluid path. The cartridge is within the device and prefilled, or the cartridge is prefilled but inserted into the disposable part before the disposable part is adhered to the skin, or the cartridge is within the device and empty (not prefilled) and the user has to fill the disposable part of the device prior to use. Optionally, the disposable part may comprise a battery source to power the drive mechanism in the reusable part.

The reusable part preferably comprises the drive mechanism, the control unit, and the electric motor. The disposable and reusable part may be releasably mechanically connected to each other via a snap fit connection, or a bayonet connection or a screw type of connection. Alternatively, the disposable and reusable part are connected by a third part, a clip or assembly member. Next to the mechanical connection there may be an electrical connection between the two parts and the electrical connection may be separate from, or integrated with, the mechanical connection.

### BRIEF DESCRIPTION OF THE DRAWINGS

The subject matter of the invention will be explained in more detail in the following text with reference to preferred exemplary embodiments which are illustrated in the attached drawings, showing:
Figure 1: Prior art device according to WO17219154
Figure 2: Patch injection device with a housing, an adhesive substrate with release liner
Figure 3: Patch injection device with a housing, an adhesive substrate, the release liner has been removed
Figure 4: Cross section of the housing, the substrate and the release liner
Figure 5: Detail showing a corner of the housing, the substrate, the cut-out and the release liner
Figure 6: Top view of the substrate and release liner
Figure 7: Detail layered substrate
Figure 8: Removal of the release liner using one hand for the liner and the other hand holding the housing.
Figure 9: Winglet on the patch injection device for skin removal

### DETAILED DESCRIPTION OF PREFERRED EMBODIMENTS

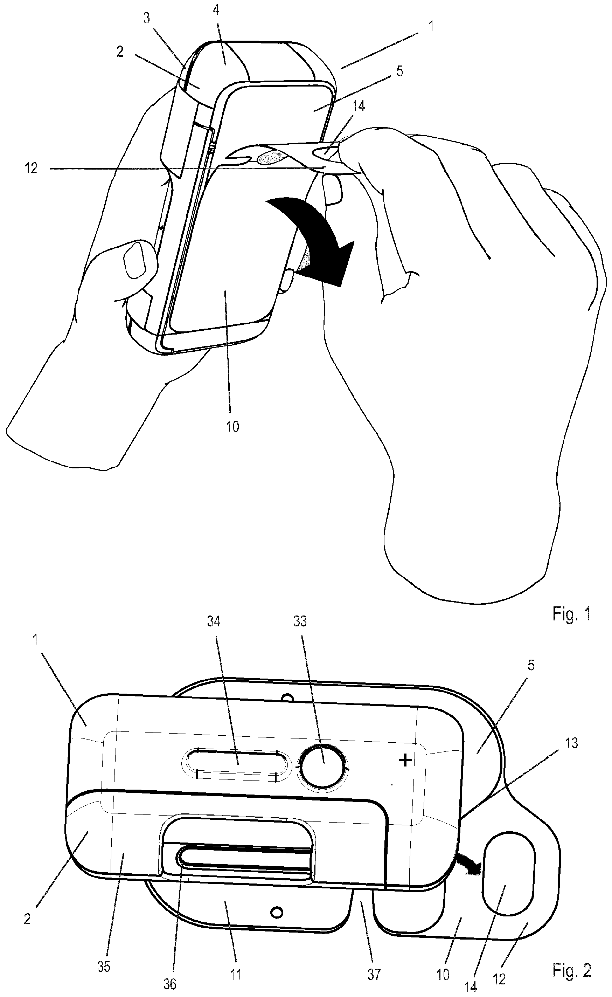

A patch injection device 1 according to the prior art (WO17219154) is shown in Figure 1. The patch injection device 1 comprises a housing 2 which can be attached to the skin of a patient using an adhesive substrate 5. The adhesive substrate 5 is covered by a removable release liner 10 which extends laterally beyond the dimensions of the housing 2 forming a pull tab 12 comprising a hole 14. The user can hold the device in one hand and grip the pull tab 12 in the hole 14 with his other hand to remove the release liner 10 in the direction indicated by the arrow. The user does not have to peel the release liner 10 directly from the substrate 5 and can hold the device while removing the release liner thus improving device handling. For large dose patch injectors, the available surface area for skin attachment is, for this example, limited to the bottom surface of a bottom housing part 4 and the weight of the device may call for stronger adhesives for skin attachment to prevent premature detachment of the device.

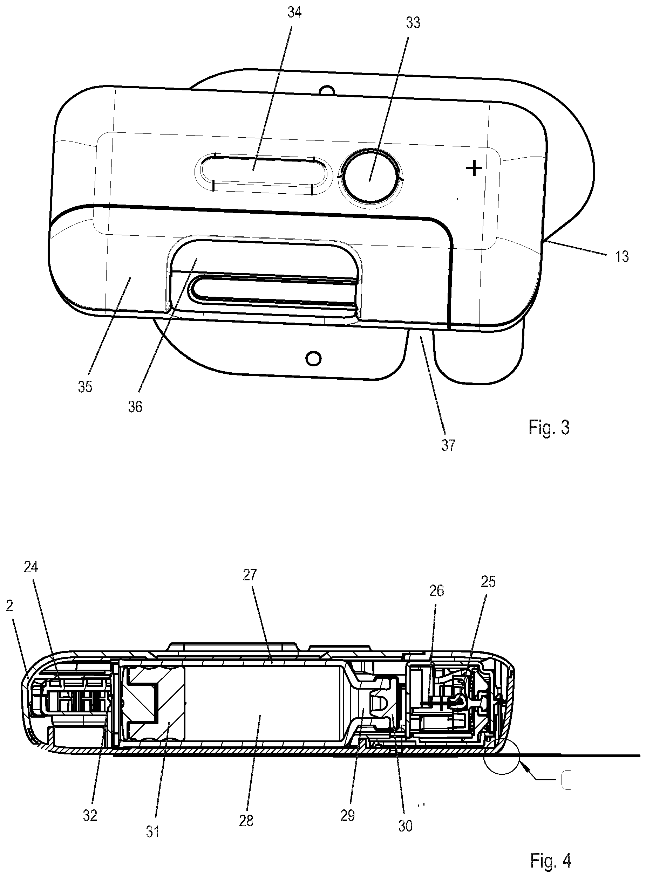

A patch injector 1 according to the present invention is shown in Figures 2 to 8. The adhesive substrate 5 at least partially surrounds the housing 2 forming a skirt 11. The adhesive substrate has a first surface contacting the release liner 10 and a second surface contacting the housing 2, more preferably the bottom housing 4. The first surface facing the liner is preferably fully covered with a skin adhesive whereas the second surface facing the housing has no adhesive in the free standing area of the skirt. The skirt 11 comprises at least one cut out 13. Drawing the virtual contour for the substrate 5 would result in a rectangular shape with four rounded corners and the cut out 13 is defined by an area of the substrate 5 that has been left out in the one corner. The removable release liner 10 is attached to the substrate (Figure 2) or has been removed (Figure 3). The removable release liner 10 comprises a pull tab 12 which extends from the housing 2 and the adhesive substrate. The pull tab 12 may have a hole 14 such that the pull tab can be easily gripped by the user. In the current example the pull tab 12 is unitarily formed with the removable release liner 10, optionally, the pull tab 12 is a separate part that is attached or connected to the release liner. Optionally, the release liner may have an arrow or sign indication the direction for withdrawing the release liner. Such an arrow or indicator may be printed or embossed on both sides of the release liner, preferably in the area of the cut out 13 for the substrate. Alternatively, the hole 14 may have a different shape, for example an "arrow shape" indicating the direction for liner removal. The indicator on the release liner preferably starts at the location where the cut-out 13 of the substrate is adjacent or close to the housing 2 or bottom housing 4.

Optionally, the substrate 5 and the release liner 10 have a second cut out 37 in skirt 11. In this case the release liner 10 follows the contours of the substrate 5 and no pull tab is formed. The second cut out is beneficial during assembly of the device, for example for sideways insertion of sterile barriers or isolation foils for battery terminals or for providing access to sterilization agents such as ETO. The second cut out 37 is therefore referred to as an assembly cut out and is independent from the above mentioned cut out 13 promoting release liner removal prior to use.

Optionally, there may be a plurality of cut outs 13 available promoting release liner removal, which may be combined with a plurality of release liners.

The patch injection device 1 (Figure 2) may further comprise an actuation button 33 and/or an optical indicator 34 (for example a LED indicator) and/or a cover 35 for closing a cartridge (holder) present within the housing. The cover 35 is preferably a removable cover and may have a viewing window 36 for viewing a cartridge present in the patch injection device.

A cross section of the patch injection device is presented in Figure 4 showing the cartridge 27 containing a fluid medicament 28 present between a moveable piston 31 and a pierceable septum 30. Optionally, the medicament is a solid medicament, for example a lyophilisate that is reconstituted prior to use. A drive mechanism 24 comprising drive train and a piston rod 32 is configured to advance the piston 31 in the cartridge towards an outlet 29 which is closed by the septum 30. The housing 2 furthermore houses a fluid path 25 comprising at least one injection needle 26. The at least one injection needle 26 is preferably connected to a tubing for fluid delivery from the cartridge towards an outlet of the fluid path. The fluid path 25 may comprise two injection needles, one for piercing the septum 30 (in the shown example needle 26) and the one for piercing the skin of the patient. Preferably one needle is aligned with the longitudinal axis of the cartridge 27 and the other needle is oriented perpendicular thereto. A not shown needle insertion mechanism is configured to move the needle 26 from a retracted position (shown in Figure 4) to an inserted position piercing the septum. The needle insertion mechanism may simultaneously move a tip of the second needle from a retracted position inside the housing through an aperture 40 in the housing and substrate to a position outside the housing.

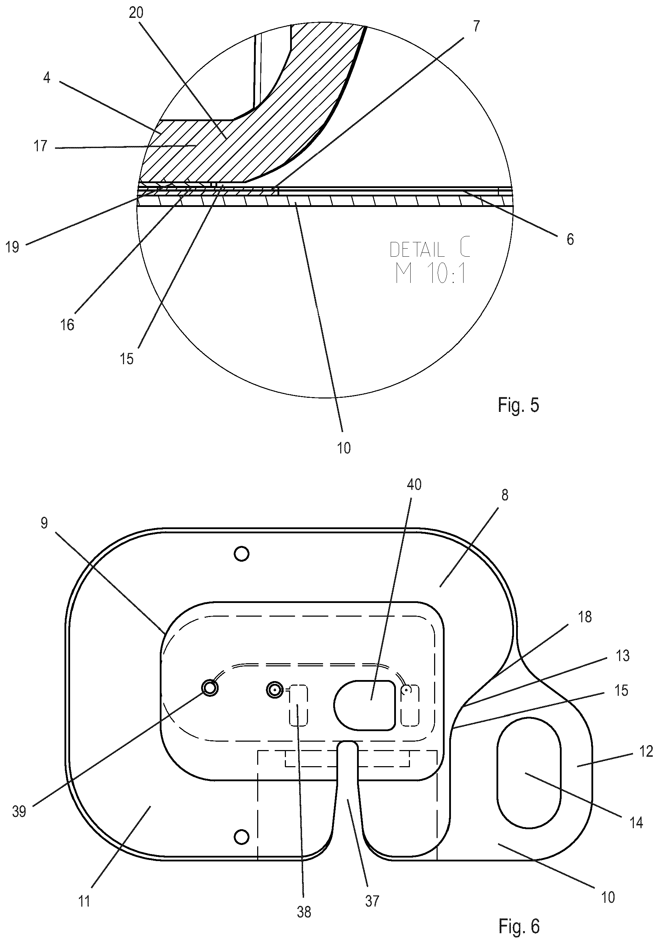

A detail of the cross section from Figure 4 is presented in Figure 5 showing the interface between a corner 20 of the bottom housing 4, the adhesive substrate 5 and the release liner 10. The cross section 17 of the housing comprises a substantially flat bottom surface being part of the contacting surface 19 between the housing and the substrate. Due to the presence of the cut out 13, the substrate 5 is also shown as a local cross section 16 (hatched) whereas the part of the substrate 5 that is out of the cross section plane is indicated as non-hatched. The cut out 13 reaches or is adjacent to the housing and this is shown as location 15. Preferably, the indication arrows on the release liner 10 start at location 15.

A top view of the substrate 5 and the release liner 10 is presented in Figure 6, showing the first surface area 8 of the substrate 5 contacting the release liner 10 and the second surface area 9 for contacting the housing. The excess area or skirt 11 surrounds the housing having the cut out 13 and a second cut out 37. The cut out 13 is formed as an arc 18 and the arc 18 comes close to or is adjacent to the second surface area 9 intended for fixation of the housing to the substrate, presented as location 15. The pull tab 12 extends from the cut out 13. The substrate may further comprise at least one sensor area 38 and /or at least one electrical contact or wiring 39. The sensor may be a proximity or contact sensor which may be a mechanical sensor or an electrical sensor, for example a capacitive sensor. Furthermore the substrate has the aperture 40 for the skin insertion needle.

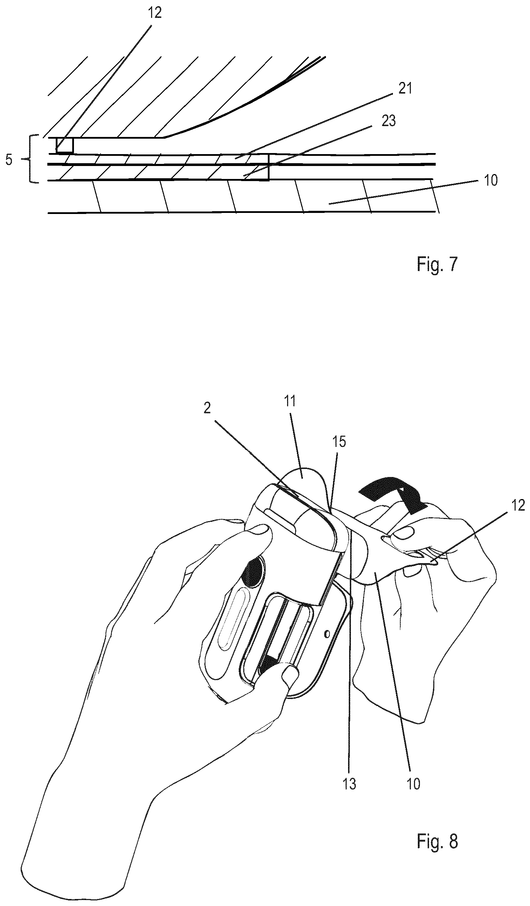

The layered substrate 5 comprises at least one adhesive layer 22 for contacting the housing and at least one supportive layer 21 and at least one skin adhesive layer 23. The supportive layer is positioned between the skin adhesive layer and the adhesive layer for contacting the housing (Figure 7). Alternatively, the substrate is based on a plurality of double sided adhesive tapes, each having a supportive layer and two adhesive layers adapted to the specific purpose, e.g. either an internal adhesive layer for mechanical support or an external adhesive layer for contacting the housing or the release liner.

The skirt 11 may be locally mechanically supported formed by the substrate itself (for example a rippled, or folded substrate structure) or the skirt is locally stiffened by adding ribs or films to the surface, preferably onto the second surface facing the housing.

The removal of the release liner is presented in Figure 8. The user holds the housing 2 of the patch injection device 1 in one hand and can grip the pull tab 12 with his other hand. The patch injection device comprises the skirt 11 of the substrate 5 surrounding the housing and the pull tab 12 is formed as an extension of the release liner 10. The cut out 13 of the substrate 5 together with the pull tab 12 ensures that the pull tab can be gripped on both sides by the user. By pulling the pull tab in the direction indicated by the arrow, the mechanical load is directed to location 15 were the cut out is adjacent to the housing. The housing, more particularly the bottom housing part provides mechanical support to the flexible substrate 5 as the substrate 5 is attached to the bottom housing. As a consequence, the peel off force releasing the liner from the substrate is directed to the housing via the substrate at the location were the cut out is adjacent to the housing. As a consequence, the release liner will first start to peel from the substrate where the substrate is mechanically supported and not in the skirt area which is not mechanically supported by the housing. The starting point for the liner removal will be location 15 and the liner removal will progress to the other areas, supported by the housing and the non-mechanically supported areas in the flexible skirt. With this configuration an easy removal of the release liner can be accomplished while holding the housing containing the (expensive) medicament.

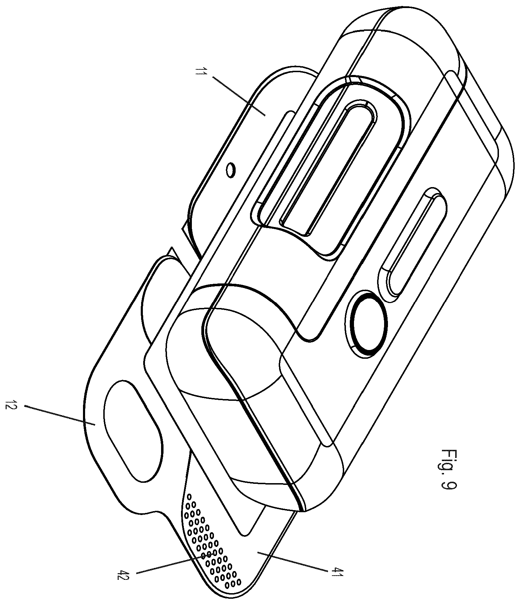

Optionally, the patch injection device may have a feature to facilitate the removal of the device from the skin after skin attachment. As an example, the adhesive substrate 5 comprises a winglet 41 (Figure 9) being part of the skirt 11 and the area on the winglet facing the release liner before liner removal is not covered by a skin adhesive whereas the remaining part of the substrate (on the first surface) is covered with a skin adhesive. In this example the winglet 41 is located next to the pulltab 12. After liner removal, the first surface of the substrate attaches to the skin except for the winglet. The winglet thus can be easily grasped by the user for removal of the device from the skin once the medication has been injected. The top surface of the winglet facing the user may comprise an indicator, for example dots 42 (Figure 9). Optionally, the dots are embossed into the substrate or printed onto the substrate to increase the roughness such that the load can be easily transferred from the user's fingers to the substrate.

### LIST OF REFERENCE SIGNS

| | | | |
|---|---|---|---|
| 1 | Patch injection device | 23 | Skin adhesive |
| 2 | Housing | 24 | Drive mechanism |
| 3 | Top housing | 25 | Fluid path |
| 4 | Bottom housing | 26 | Injection needle |
| 5 | Adhesive substrate | 27 | Cartridge |
| 6 | First surface | 28 | Fluid medicament |
| 7 | Second surface | 29 | Outlet |
| 8 | First surface area | 30 | Septum |
| 9 | Second surface area | 31 | Piston |
| 10 | Removable release liner | 32 | Piston rod |
| 11 | Skirt | 33 | Actuation button |
| 12 | Pull tab | 34 | Optical indicator |
| 13 | Cut out | 35 | Cover |
| 14 | Hole | 36 | Viewing window |
| 15 | Location where cut out reaches housing | 37 | Second cut out |
| 16 | Cross section substrate | 38 | Sensor area |
| 17 | Cross section housing | 39 | Electrical contacts / wiring |
| 18 | Arc | 40 | Aperture |
| 19 | Contacting surface housing- substrate | 41 | Winglet |
| 20 | Corner housing | 42 | Dots |
| 21 | Supportive layer | | |
| 22 | Adhesive layer to housing | | |

## Claims

1. A patch injection device comprising:
a housing (2),
an adhesive substrate (5) having a first surface (6) with a first surface area (8) for skin attachment and a second surface (7) which is opposite the first surface (6) attaching partly to the housing (2),
a removable release liner (10),
wherein the surface area of the second surface attaching to the housing (9) is smaller than the first surface area (8) and wherein the excess area defines a skirt (11) of the substrate at least partially surrounding the housing (2),
and the first surface (6) of the substrate attaches to the removable release liner (10) which fully covers the first surface area (8) of the substrate,
**characterized by** a pull tab (12) that is part of the release liner (10) and which is located in a cut out (13) of the skirt (11) of the substrate (5), the cut out (13) being adjacent or locally congruent to the second surface (7) contacting the housing to the substrate (5) and wherein the housing (2) comprises at least one corner (20) and the at least one corner (20) is adjacent to the cut out (13) of the skirt (11) such that a peel force is transmittable from the release liner (10) to the housing (2) at the location where the cut-out reaches the housing.

2. The patch injection device according to claim 1 wherein the release liner (10) partially extends beyond the adhesive substrate (5) , preferably in the area of the cut-out (13), such that the surface area of the release liner is larger than the first surface area (8).

3. The patch injection device according to claims 1 or 2 wherein the adhesive substrate (5) is flexible.

4. The patch injection device according to any of the previous claims wherein the pull tab (12) is integrally formed with the release liner (10) or the pull tab (12) is a separate part attached to the release liner (10).

5. The patch injection device according to any of the previous claims wherein the pull tab (10) comprises a hole.

6. The patch injection device according to any of the previous claims wherein the peel force is transmittable to the housing (2) via the adhesive substrate (5) at the location where the cut out (13) reaches the housing.

7. The patch injection device according to any of the previous claims wherein the cut-out (13) of the skirt (11) is arc shaped.

8. The patch injection device according to any of the previous claims wherein the release liner (10) is split into two separate parts each having a separate pull tab.

9. The patch injection device according to any of the previous claims wherein the adhesive substrate (5) is partially stiffened, preferably in the skirt (11) surrounding the housing.

10. The patch injection device according to any of the previous claims wherein the adhesive substrate (5) is a multilayered substrate comprising at least one supportive layer (21) and at least two adhesive layers (22, 23) for attaching the housing (2) to a part of the second surface (7) and for attaching the removable release liner (10) to the first surface (6).

11. The patch injection device according to any of the previous claims wherein the housing (2) is an outside housing comprising a top housing part (3) and a bottom housing part (4) and wherein the adhesive substrate (5) is attached to the bottom housing part (4).

12. The patch injection device according to any of the previous claims wherein the housing (2) encloses a drive mechanism (24) for expelling a medicament (28), a control unit for controlling medicament delivery and a fluid path (25) comprising an injection needle (26) for delivery of the medicament.

13. The patch injection device according to claim 12 wherein the housing (2) houses a cartridge (27) comprising a fluid medicament.

## Patentansprüche

1. Patch-Injektionsvorrichtung, umfassend:
ein Gehäuse (2),
ein Klebesubstrat (5), welches eine erste Oberfläche (6) mit einem ersten Oberflächenbereich (8) zur Befestigung auf der Haut und eine zweite Oberfläche (7) aufweist, die der ersten Oberfläche (6) gegenüberliegt und teilweise an dem Gehäuse (2) befestigt ist,
eine entfernbare Trennfolie (10),
wobei der Oberflächenbereich (9) der zweiten Oberfläche, der an dem Gehäuse befestigt ist, kleiner ist als der erste Oberflächenbereich (8) und wobei der überschüssige Bereich eine Schürze (11) des Substrats definiert, die das Gehäuse (2) zumindest teilweise umgibt,
und die erste Oberfläche (6) des Substrats an der entfernbaren Trennfolie (10) befestigt ist, welche den ersten Oberflächenbereich (8) des Substrats vollständig bedeckt,
**gekennzeichnet durch** eine Abziehlasche (12), welche Teil der Trennfolie (10) ist und welche in einem Ausschnitt (13) der Schürze (11) des Substrats (5) angeordnet ist, wobei sich der Ausschnitt (13) angrenzend zu der zweiten Oberfläche (7) befindet oder lokal deckungsgleich damit ist und das Gehäuse mit dem Substrat (5) in Kontakt bringt und wobei das Gehäuse (2) mindestens eine Ecke (20) umfasst und sich die mindestens eine Ecke (20) angrenzend zu dem Ausschnitt (13) der Schürze (11) befindet, so dass an der Stelle, wo der Ausschnitt das Gehäuse erreicht, eine Abziehkraft von der Trennfolie (10) auf das Gehäuse (2) übertragbar ist.

2. Patch-Injektionsvorrichtung nach Anspruch 1, wobei die Trennfolie (10) teilweise über das Klebesubstrat (5) hinausragt, vorzugsweise im Bereich des Ausschnitts (13), so dass der Oberflächenbereich der Trennfolie größer ist als der erste Oberflächenbereich (8).

3. Patch-Injektionsvorrichtung nach Anspruch 1 oder 2, wobei das Klebesubstrat (5) flexibel ist.

4. Patch-Injektionsvorrichtung nach einem der vorstehenden Ansprüche, wobei die Abziehlasche (12) einstückig mit der Trennfolie (10) ausgebildet ist oder die Abziehlasche (12) ein separates Teil ist, das an der Trennfolie (10) befestigt ist.

5. Patch-Injektionsvorrichtung nach einem der vorstehenden Ansprüche, wobei die Abziehlasche (10) ein Loch umfasst.

6. Patch-Injektionsvorrichtung nach einem der vorstehenden Ansprüche, wobei die Abziehkraft an der Stelle, wo der Ausschnitt (13) das Gehäuse erreicht, über das Klebesubstrat (5) auf das Gehäuse (2) übertragbar ist.

7. Patch-Injektionsvorrichtung nach einem der vorstehenden Ansprüche, wobei der Ausschnitt (13) der Schürze (11) bogenförmig ist.

8. Patch-Injektionsvorrichtung nach einem der vorstehenden Ansprüche, wobei die Trennfolie (10) in zwei separate Teile aufgeteilt ist, welche jeweils eine separate Abziehlasche aufweisen.

9. Patch-Injektionsvorrichtung nach einem der vorstehenden Ansprüche, wobei das Klebesubstrat (5) teilweise versteift ist, vorzugsweise in der Schürze (11), die das Gehäuse umgibt.

10. Patch-Injektionsvorrichtung nach einem der vorstehenden Ansprüche, wobei das Klebesubstrat (5) ein mehrschichtiges Substrat ist, welches mindestens eine Trägerschicht (21) und mindestens zwei Klebeschichten (22, 23) zum Befestigen des Gehäuses (2) an einem Teil der zweiten Oberfläche (7) und zum Befestigen der entfernbaren Trennfolie (10) an der ersten Oberfläche (6) umfasst.

11. Patch-Injektionsvorrichtung nach einem der vorstehenden Ansprüche, wobei das Gehäuse (2) ein Außengehäuse ist, welches einen oberen Gehäuseteil (3) und einen unteren Gehäuseteil (4) umfasst, und wobei das Klebesubstrat (5) an dem unteren Gehäuseteil (4) befestigt ist.

12. Patch-Injektionsvorrichtung nach einem der vorstehenden Ansprüche, wobei das Gehäuse (2) einen Antriebsmechanismus (24) zum Ausstoßen eines Medikaments (28), eine Steuereinheit zum Steuern der Medikamentabgabe und einen Fluidpfad (25), der eine Injektionsnadel (26) zur Abgabe des Medikaments umfasst, umschließt.

13. Patch-Injektionsvorrichtung nach Anspruch 12, wobei das Gehäuse (2) eine Kartusche (27) beherbergt, welche ein fließfähiges Medikament umfasst.

## Revendications

1. Dispositif d'injection par patch comprenant :
un boîtier (2),
un substrat adhésif (5) ayant une première surface (6) avec une première zone de surface (8) destinée à être fixée sur la peau et une seconde surface (7) qui est opposée à la première surface (6) se fixant partiellement au boîtier (2),
une feuille anti-adhésive amovible (10),
dans lequel la zone de surface de la seconde surface se fixant au boîtier (9) est inférieure à la première zone de surface (8) et dans lequel la zone excédentaire définit une jupe (11) du substrat entourant au moins partiellement le boîtier (2),
et la première surface (6) du substrat se fixe à la feuille anti-adhésive amovible (10) qui couvre entièrement la première zone de surface (8) du substrat,
**caractérisé par** une languette à tirer (12) qui fait partie de la feuille anti-adhésive (10) et qui est située dans une découpe (13) de la jupe (11) du substrat (5), la découpe (13) étant adjacente ou localement congruente à la seconde surface (7) mettant en contact le boîtier avec le substrat (5) et dans lequel le boîtier (2) comprend au moins un coin (20) et l'au moins un coin (20) est adjacent à la découpe (13) de la jupe (11) de telle sorte qu'une force de pelage est transmissible de la feuille anti-adhésive (10) au boîtier (2) au niveau de l'emplacement où la découpe atteint le boîtier.

2. Dispositif d'injection par patch selon la revendication 1, dans lequel la feuille anti-adhésive (10) s'étend partiellement au-delà du substrat adhésif (5), de préférence dans la zone de la découpe (13), de telle sorte que la zone de surface de la feuille anti-adhésive est plus grande que la première zone de surface (8).

3. Dispositif d'injection par patch selon les revendications 1 ou 2, dans lequel le substrat adhésif (5) est souple.

4. Dispositif d'injection par patch selon l'une quelconque des revendications précédentes, dans lequel la languette à tirer (12) est formée d'un seul tenant avec la feuille anti-adhésive (10), ou la languette à tirer (12) est une pièce séparée fixée à la feuille anti-adhésive (10).

5. Dispositif d'injection par patch selon l'une quelconque des revendications précédentes, dans lequel la languette à tirer (10) comprend un trou.

6. Dispositif d'injection par patch selon l'une quelconque des revendications précédentes, dans lequel la force de pelage est transmissible au boîtier (2) par l'intermédiaire du substrat adhésif (5) au niveau de l'emplacement où la découpe (13) atteint le boîtier.

7. Dispositif d'injection par patch selon l'une quelconque des revendications précédentes, dans lequel la découpe (13) de la jupe (11) est en forme d'arc.

8. Dispositif d'injection par patch selon l'une quelconque des revendications précédentes, dans lequel la feuille anti-adhésive (10) est divisée en deux parties distinctes ayant chacune une languette à tirer séparée.

9. Dispositif d'injection par patch selon l'une quelconque des revendications précédentes, dans lequel le substrat adhésif (5) est partiellement raidi, de préférence dans la jupe (11) entourant le boîtier.

10. Dispositif d'injection par patch selon l'une quelconque des revendications précédentes, dans lequel le substrat adhésif (5) est un substrat multicouche comprenant au moins une couche de support (21) et au moins deux couches adhésives (22, 23) pour la fixation du boîtier (2) à une partie de la seconde surface (7) et pour la fixation de la feuille anti-adhésive amovible (10) à la première surface (6).

11. Dispositif d'injection par patch selon l'une quelconque des revendications précédentes, dans lequel le boîtier (2) est un boîtier extérieur comprenant une partie supérieure de boîtier (3) et une partie inférieure de boîtier (4), et dans lequel le substrat adhésif (5) est fixé à la partie inférieure de boîtier (4).

12. Dispositif d'injection par patch selon l'une quelconque des revendications précédentes, dans lequel le boîtier (2) renferme un mécanisme d'entraînement (24) permettant d'expulser un médicament (28), une unité de commande permettant de commander une administration de médicament et un chemin de liquide (25) comprenant une aiguille d'injection (26) permettant l'administration du médicament.

13. Dispositif d'injection par patch selon la revendication 12, dans lequel le boîtier (2) loge une cartouche (27) comprenant un médicament liquide.
